# EUROPEAN PATENT SPECIFICATION

(11) **EP 1 204 737 B1**
(45) Date of publication and mention of the grant of the patent: **25.10.2006**
(21) Application number: 00945465.3
(22) Date of filing: 27.07.2000
(51) Int. Cl.: C12M 1/34, C12Q 1/06, C12Q 1/24, G01N 33/52, G01N 33/53, G01N 33/533, G01N 33/569, G01N 33/58, G01N 33/96, G01N 37/00

(54) **INTERNAL QUALITY CONTROL**
INTERNE QUALITÄTSKONTROLLE
CONTROLE DE QUALITE INTERNE

(30) Priority: 02.08.1999 AU PP196199
(43) Date of publication of application: 15.05.2002
(73) Proprietor: BTF Pty Ltd., Fairlight, NSW 2094 (AU)
(72) Inventor: VESEY, Graham, Gladesville, NSW 2111 (AU)
(74) Representative: Flaccus, Rolf-Dieter
(86) International application number: PCT/AU2000/000896
(87) International publication number: WO 2001/009281

(56) References cited:
- WO-A-97/08204
- GB-A- 2 057 685
- US-A- 5 741 662
- VESEY G ET AL: "Application of flow cytometric methods for the routine detection of Cryptosporidium and Giardia in water" CYTOMETRY, ALAN LISS, NEW YORK, US, vol. 16, no. 1, 1 May 1994 (1994-05-01), pages 1-6, XP002144083 ISSN: 0196-4763
- NIEMINSKI E C ET AL: "Comparison of two methods for detection of Giardia cysts and Cryptosporidium oocysts in water." APPLIED AND ENVIRONMENTAL MICROBIOLOGY. UNITED STATES MAY 1995, vol. 61, no. 5, May 1995 (1995-05), pages 1714-1719, XP002268703 ISSN: 0099-2240
- HASHIMOTO ATSUSHI ET AL: "Prevalence of Cryptosporidium oocysts and giardia cysts in the drinking water supply in Japan." WATER RESEARCH. ENGLAND FEB 2002, vol. 36, no. 3, February 2002 (2002-02), pages 519-526, XP002268704 ISSN: 0043-1354
- LECHEVALLIER M W ET AL: "Evaluation of the immunofluorescence procedure for detection of Giardia cysts and Cryptosporidium oocysts in water." APPLIED AND ENVIRONMENTAL MICROBIOLOGY. UNITED STATES FEB 1995, vol. 61, no. 2, February 1995 (1995-02), pages 690-697, XP002268705 ISSN: 0099-2240

## Description

This invention relates to the field of internal quality control in diagnostic analysis, particularly in the analysis of microorganisms or other small particles, such as the detection of bacteria, protozoa, yeast, fungi, viruses, prions and the like, in water or other potable fluids, food, blood, urine, faeces, cerebro spinal fluid, and the like.

Note: References are collected at the end of the specification.

### Background of the Invention

Water is routinely tested for the presence of *Cryptosporidium* and *Giardia.* Detection relies on immunofluorescence techniques to stain cysts and oocysts with a green fluorescent dye. The stained sample is examined using epifluorescent microscopy and the number of green fluorescing cysts and oocysts recorded.

The methodology involves numerous processes in which cysts and oocysts can be lost. It is, therefore, important to perform stringent quality control to monitor the performance of the methodology. At present, quality control is an external process that involves analysing a standard that contains a known number of cysts and oocysts. Such a quality control test is typically performed after analysing 10 or 20 samples. This external quality control is far from ideal. Losses of cysts and oocysts can very greatly between different samples. Some samples may be unsuitable for analysis. Furthermore, if the quality control result is poor then all results from the 10 or 20 samples must be discarded.

The same situation applies to diagnostic analysis in a wide variety of fields, where an external quality control is subject to the relevant diagnostic analysis being applied as a measure of performance/accuracy.

A need accordingly exists for accurate, convenient, cost-effective, and repeatable quality control in diagnostic analysis, which is currently not met by the prior art.

### Summary of Invention

In accordance with a first aspect of this invention there is provided a diagnostic internal control method comprising providing a population of bioparticles, modifying the bioparticles to permanently and detectably tag each bioparticle to give tagged bioparticles, forming an internal quality control sample of a defined quanta of tagged bioparticles in a defined volume of carrier, adding the control sample to an assay sample for the detection of the same bioparticles, carrying out said assay, and thereafter determining the precise number of tagged bioparticles detected in the assay which on comparison with the quanta of added tagged bioparticles provides a determination of assay accuracy.

In accordance with a further aspect of this invention there is provided a method for providing an internal quality control in the analysis of bioparticles in potable fluids, which method comprises providing a sample of the bioparticles, subjecting the sample to detectable tagging, forming an internal control sample of a defined quanta of tagged bioparticles in a determined volume, adding said internal control sample to a test sample and subjecting the sample to analysis and determining the proportion of tagged bioparticles within the sample, wherein comparing the number of detected tagged test bioparticles compared with the known quanta of tagged bioparticles added to the sample provides a measure of assay accuracy and gives actual numbers allowing for losses in performing the assay.

In accordance with a further aspect of this invention there is provided an internal quality control standard which comprises a predetermined quanta of bioparticles, said bioparticles being of the type subject to routine analysis, said predetermined quanta of bioparticles being tagged with a detectable tag, and provided in a carrier of defined volume.

### Detailed Description of the Invention

The accuracy of diagnostic assays is critical to their performance as a tool in modem analysis. Plainly, an assay which does not give accurate results is of no real benefit as it may understate or overstate the presence of the specific entity being measured. Incorrect analysis may give rise to inappropriate decisions being made and these may have significant deleterious effects.

This invention is concerned with internal quality control, that is providing internal standards of tagged bioparticles which are added to a sample and subject to routine analysis, whereafter the number of tagged bioparticles in the sample, when compared with the known number of bioparticles added to the sample, is a direct measure of assay accuracy.

In accordance with a first aspect of this invention there is provided a diagnostic internal control method comprising providing a population of bioparticles, modifying the bioparticles to permanently and detectably tag each bioparticle to give tagged bioparticles, forming an internal quality control sample of a defined quanta of tagged bioparticles in a defined volume of carrier, adding the control sample to an assay sample for the detection of the same bioparticles, carrying out said assay, and thereafter determining the absolute number of tagged bioparticles detected in the assay which on comparison with the quanta of added tagged bioparticles provides a determination of assay accuracy.

The bioparticles may be a microorganism, for example selected from a bacteria, fungus, yeast or virus, or may be a single or multi-cellular protozoa, a prion or other bioparticle. Examples of such bioparticles include *Cryptosporidium, Giardia, Cyclospora, Toxoplasma, Eimeria, Legionella, Samonella, Leptospirosis, Escherichia, Saccharomyces, Clostridium, Vibrio, Pseudomonas, Anthrax,* blood cells, HIV, Norwalk virus, herpes simplex virus.

The assay sample for the detection of bioparticles, may be water or another potable fluid such as fruit juice, wine, beer, milk, cider or the like. The sample may be a food, such as poultry, beef, eggs, cheese, preserved meats such as salami or ham and the like. The sample may be in the form of blood, cerebro spinal fluid, urine, tissue extracts, faeces, and the like.

Bioparticles which comprise the control bioparticles are detectably tagged with a tag which alters the chemical and/or physical properties of the bioparticle in a manner which is readily detectable. An example of ready detectability is via microscopic analysis, where tags such as fluorophores may be used. When shone with light at an appropriate wavelength, fluorophores fluoresce thus identifying bioparticles tagged with the fluorophores. Non-tagged bioparticles do not fluoresce under such conditions. Other readily detectable tags which effect the physical and/or chemical properties of labelled bioparticles, such as density, shape or appearance include colloidal compounds such as colloidal gold, labelling with electron dense agents such as ferritin or other iron or heavy metal compounds/complexes and the like. Other tags which may be used include radiolabelling using a radioactive isotope, for example which tags cell surface protein, lipids or carbohydrates or internal such species, and modification of the DNA or RNA to alter the chemical and/or physical properties of the bioparticle. The latter may involve incorporation of a gene that causes luminescence (Catrin et al., 1999), fluorescence (Nobuhide Doi & Hiroshi Yanagawa, 1999) or other well established gene expression systems such as antibiotic resistance, chemical tolerance, antigen expression, enzyme expression or a change in metabolic activity.

Detectable tags may tag cell surface proteins, lipids, glycolipids, and/or carbohydrate, or internal such species within the bioparticle. Where tags bind permanently to cell surface or intracellular proteins, lipids and/or carbohydrates various advantages arise. For example, such cells do not need to be permobilised or changed in any physical way for labelling to occur, the tagging is permanent, and damage or disruption of cells will not cause the signal from the detectable tag to be lost.

DNA or RNA within a bioparticle may be tagged in a permanent or non-permanent manner, for example using nucleic acid reactive fluorochromes which form a permanent bond with DNA or RNA, or DNA/RNA intercollating fluorochromes which do not form a permanent bond with DNA or RNA. DNA/RNA tagging may not be as advantageous as other forms of tagging mentioned above due to DNA/RNA turnover/degradation which may cause the tag to be lost or diminished in detectable signal.

Methods for detectably tagging or labelling bioparticles, such as microorganisms, yeast cells, viruses and the like are well established, see for example Gröndahl *et al.,* 1997 and Chaka *et al.,* 1995. Fluorescently stained yeast and bacteria are an item of commerce, being sold by various companies, including Molecular Probes Inc of Eugene, USA. By way of example, cells to be labelled with a fluorescent marker may simply be added to a solution of the fluorophore such that proteins or other species on the cell surface, or on the inside of the cell, are fluorescently labelled. Fluorochromes are generally provided with one or more functional groups which allow coupling for example to protein functional groups such as the ε-amino group of lysine.

In like manner, colloidal agents, electron dense agents and the like generally contain one or more reactive groups or are derivatised to contain one or more reactive groups to allow labelling, for example with proteinaceous functional groups, such as the ε-amino group of lysine, or the carboxylic acid functionality of glutamic acid.

The DNA or RNA of a microorganism may be altered to include a DNA sequence which produces a physical or chemical change in the microorganism, detectable for example by change of shape, density, morphological appearance and the like using established molecular biology techniques a are known in the art (see, for example, Catrin et al., 1999; Nobuhide Doi & Hiroshi Yanagawa, 1999).

Where in a routine assay different types of bioparticles are analysed, the control sample may contain a predetermined number of each type of bioparticle, with each type of bioparticle being tagged in a manner which allows ready identification of each type of tagged bioparticle. An example would be to tag each type of bioparticle with a different type of fluorescently active agent. For example a wide variety of dyes are commercially available which fluoresce green, red, yellow, orange or blue, such as Fluorescein or Rhodamine (Haugland 1998).

Bioproperties are inactivated either prior to or after tagging to provide stability carried out by established techniques such as X-ray inactivation, snap freezing, chemical inactivation and the like. The inactivation does not effect the chemical or physical properties of the bioparticle, such that in assay it behaves in the same manner as bioparticles being tested in assay conditions.

Tagged bioparticles are detected within the process of analysis, hence the control is an internal control. The type of routine analysis depends on the analysis system used in the specific diagnostic assay being carried out. By way of example, water quality may be determined by microscopic analysis of a water sample under high magnification where the different types of bioparticles, such as microorgansims and protozoans, can be identified.

Other diagnostic assays may involve flow cytometry where cells can be readily sorted according to their physical properties (Shapiro, 1995). The assay of bioparticles, such as microorganisms, in water or other potable fluids, in food analysis, and in analysis of biological fluids such as blood, sputum, cerebro spinal fluid, faecal material and the like, may be conducted by way of microscopic analysis, flow cytometry, cytometry, laser scanning cytometry, confocal microscopy or other well established analytical techniques as routinely used in water and feed analysis, medical diagnostic tests, cell culture, tissue culture infectivity and animal infectivity analysis. Also, immunological detection methods such as enzyme linked immunoabsorbant assays (ELISA), colour-metric detection and imunofluorescence, or nucleic acid detection methods such as DNA or RNA probe hybridisation, fluorescence in-situ hybridisation (FISH) or polymerase chain reaction (PCR) may be used.

Whatever type of assay is carried out for the detection of bioparticles, the control sample of a predetermined quanta of bioparticles is added to the assay, the assay is carried out, and the determination of the proportion of tagged bioparticles so detected in the assay sample is made and compared with the predetermined number of tagged bioparticles added, as a measure of assay accuracy. This may be referred to as enumeration, a characteristic feature of this invention, where the precise number of tagged bioparticles in a control sample is known/predetermined, and on addition of the control sample to an assay, are precisely or absolutely determined as a measure of assay efficiency. The measure of assay efficiency is then used to calculate the number of test organisms present in the sample. Particularly, the number of tagged bioparticles detected when compared with the quanta added to the assay gives a percentage recovery, for example if 10 tagged bioparticles are added to an assay for the same bioparticle and 8 tagged bioparticles are detected on analysis the percentage recovery is 80%. This is a direct measure of the percentage recovery/detection of untagged particles in the assay.

In accordance with a further aspect of this invention there is provided a method for providing an internal quality control in the analysis of bioparticles in potable fluids, which method comprises providing a sample of bioparticles, subjecting the sample to detectable tagging, forming an internal control sample of a defined quanta of tagged bioparticles in a determined volume, adding said internal control sample to a test sample and subjecting the sample to analysis and determining the proportion of tagged bioparticles within the sample, wherein comparing the number of detected tagged test bioparticles compared with the known quanta of tagged bioparticles added to the sample provides a measure of assay accuracy.

Preferably, the potable fluid is water, and examples of bioparticles which may be detected include *Cryptosporidium* and *Giardia.*

In a further aspect of this invention there is provided an internal quality control standard which comprises a predetermined quanta of bioparticles, said bioparticles being of the type subject to routine analysis, said predetermined quanta of bioparticles being tagged with the detectable tag, and provided in a carrier of defined volume.

Bioparticles which are tagged as previously described may be formed into a predetermined number in a predetermined volume of carrier, for example by way of flow cytometry where cells are separated based on their physical properties (Shapiro, 1995; Vesey *et al.,* 1994), or by other standard techniques in the art.

The tagged bioparticles in defined quanta and volume may be provided in a container which allows for ready addition of said tagged bioparticles to an assay sample.

The present invention has wide applicability in testing samples for the presence of bioparticles, for example microorganisms. One specific area of importance is in quality control in relation to water testing. Potable water is generally tested for the presence of microorganisms, in particular, the presence of the organisms *Cryptosporidium* and *Giardia.* Detection relies on immunofluoresence techniques to stain cysts and oocysts with a green fluorescent dye. The stained sample is examined using epifluorescent microscopy and the number of green fluorescing cysts and oocysts recorded. As mentioned above it is important to perform stringent quality control procedures to monitor the performance of the methodology. The present invention enables monitoring the quality of analysis of every sample which is analysed. Water samples may have added to them known numbers of tagged cysts and oocysts, for example which have been tagged with a blue fluorescent dye. When the example is examined for green fluorescing cysts and oocysts, further examination is performed to determine if the cysts or oocysts are an internal standard. The cysts and oocysts may be simply examined to determine if they are fluorescing blue as well as green. Any cysts or oocysts detected at fluoresce green but not blue are not an internal standard. In this approach, various fluorescent dyes may be used which distinguish the labelled microorganisms. Other labelling techniques as described above may of course be utilised.

In the water quality area, it is convenient to inactive cysts and oocysts with gamma radiation prior to conjugation with a fluorescent dye or other labelling agent. The organisms which are tagged are generally inactivated as discussed above. Flow cytometry is generally used to accurately dispense a known number of labelled cysts and oocysts into a test tube. The test tubes may be sealed and irradiated with, for example, gamma irradiation to sterilise the sample and increase shelf life. The sample may be stored at low temperature, for example 4°C until used in a standard assay for water quality.

The invention described herein allows the accurate enumeration of the internal control bioparticles, for example control cells. This enumeration which is plainly distinct from the prior art is essential as it is critical that the precise or absolute number of test organisms in a sample be measured to enable an assessment of assay accuracy.

As previously mentioned, this invention has wide applicability in the detection of microorganisms or other bioparticles, for example, the detection of bacteria, protozoa, yeast, fungi or viruses in food, water or other potable liquids, blood, urine, faeces, food etc.

This invention will now be described by reference to the following non-limiting examples.

### Example 1

### Cryptosporidium and Giardia

*Cryptosporidium parvum* oocysts are purified from pooled faeces of naturally infected neonatal calves in Sydney. Faecal samples are centrifuged (2000g, 10 min) and resuspended in water twice and then resuspended in 5 volumes of 1% (w/w) NaHCO₃. Fatty substances are then extracted twice with 1 volume of ether, followed by centrifugation (2000g for 10 min). Pellets are resuspended in water and filtered through a layer of pre-wetted non-adsorbent cotton wool. The eluate is then overlaid onto 10 volumes of 55% (w/v) sucrose solution and centrifuged (2000g for 20 min). Oocysts are collected from the sucrose interface and the sucrose flotation step repeated until no visible contaminating material could be detected. Purified oocysts are surface sterilised with ice cold 70% (v/v) ethanol for 30 min, washed once in phosphate buffered saline (150 m mol 1⁻¹ NaCl, 15 m mol 1⁻¹ KH₂PO₄, 20 m mol 1⁻¹ Na₂HPO₄, 27 m mol 1⁻¹ KCl, pH 7.4) (PBS), and diluted in PBS to a concentration of approximately 1 x 10⁷ oocysts ml⁻¹ and stored at 4°C.

*Giardia lamblia* cysts were purchased from Waterborne Inc (New Orleans, USA).

### Conjugation of fluorochromes to cysts and oocysts

Some samples of cysts and oocysts are frozen (-80°C) to disrupt the cyst and oocyst walls prior to conjugation.

Aliquots (200µl) of cysts and oocysts (containing approximately 1 x 10⁷ are centrifuged at 12000g for 2 minutes and the supernatant removed and discarded. Cysts and oocysts are resuspended in 0.1M sodium bicarbonate pH 8.3. This washing procedure was repeated twice.

The blue fluorochrome Alexa 350 carboxylic acid, succinimidyl ester (Alexa 350 - Molecular Probes, Eugene, USA) is dissolved in dimethylsulfoxide (DMNSO) at a concentration of 1 mg per ml.

Aliquots (10 µl) of the fluorochrome/DMSO mixture are added to 200µl of the cysts and oocyst suspensions. Samples are mixed thoroughly and incubated at room temperature for 30 minutes. Samples are then centrifuged at 12000g for 2 minutes and the supernatant removed and discarded. Cysts and oocysts are resuspended in 0.1M sodium bicarbonate. This washing procedure is repeated twice.

### Aliquoting

Labelled cysts and oocysts are accurately dispensed into 6 ml plastic test tubes using flow cytometry. A Becton Dickinson FACScalibur flow cytometer is used to analyse labeled cysts and oocysts. Fluorescence and or light scatter properties of cysts or oocysts are defined and these properties used to sort 100 labeled cysts and oocysts from a sample of labeled cysts and oocysts. Sorted cysts and oocysts are dispensed into a test tube.

It is important that all cysts and oocysts that are sorted are labeled. If an unlabelled cyst or oocyst ends up in the sample then false positive results would occur.

Quality control is performed to ensure that accurate numbers of cysts and oocysts are dispensed. In this regard a proportion of test tubes are analysed using microscopy or flow cytometry to enumerate cysts and oocysts. Test tubes are weighed and tubes of an outlying weight would be discarded.

### Seeding water samples with control material

Samples of water are either collected in containers and shipped to a laboratory for concentration or samples concentrated at the sampling location. Samples that are shipped to laboratories for concentration normally range in volume from 10 to 100 litres. The sample is seeded with the control sample using the following seeding method:
1) Add 2 ml of 0.05% (v/v) tween 80 to the tube of control material.
2) Replace cap and shake vigorously.
3) Remove cap and pour control material into sample.
4) Add 3ml of 0.05% (v/v) tween 80 to the control material tube.
5) Replace cap and shake vigorously.
6) Remove cap and pour control material into sample.

Repeat steps 4, 5 and 6.

The sample is concentrated using conventional methods such as membrane filtration, cartridge filtration, pleated membrane cartridge filtration, flocculation or vortex flow filtration (Anon, 1999; Vesey *et al.,* 1994).

If the water sample is concentrated at the sample site then a portable concentration method such as cartridge filtration is employed. The filter cartridge should be seeded with the control material before concentration of the sample. The seeding method described above is suitable for seeding the filter cartridge.

Manufacturers of cartridge filters may seed filters during the manufacturing process.

Filters that have been used to concentrate water samples are returned to the analysis laboratory and the particulate material collected within the filter is eluted and further concentrated

### Analysis

Concentrated samples are treated to remove contaminating particles such as algae and mineral particles. Methods such as floatation, immunomagnetic separation (IMS) and flow cytometry are routinely used for purifying cysts and oocysts from water concentrates (Vesey *et al.,* 1994; Anon, 1999).

Purified samples are then transferred to a glass microscope slide or onto a small (13 mm) membrane filter. The slide or the membrane is stained with fluorescein isothiocyanate (FITC) stained monoclonal antibodies, incubated and washed and examined using epifluorescence microscopy (Vesey *et al.,* 1993; Anon, 1999), for example using a Nikon Optiphot2 epifluorescence microscope fitted with x 12 eyepieces and a x 20 objective (Fluor20) for examination of samples. A DM510 filter block is used for the examination of fluorochrome fluorescein isothiocyanate FITC labelled samples. The membrane or slide would be carefully scanned and the cysts and oocysts detected.

When a green fluorescing cysts or oocysts is detected it would be examined using different optical filters to determine if it was fluorescing blue thus identifying it as a control cyst or oocyst. A DM450 filter block is used for the detection of the blue fluorescence from Alexa 350 labeled control cysts and oocysts.

Once the entire membrane has been scanned the total number of cysts and oocysts detected in the sample is compared with the number of cysts and oocysts detected that fluoresce blue. These figures are then used to calculate the recovery efficiency and the actual number of cysts and oocysts present in the water sample. In one example, the sample is seeded with 100 blue control cysts and 100 blue control oocysts. Analysis of the sample results in the detection of 50 blue cysts and 30 non-blue cysts and 50 blue oocysts and 10 non-blue oocysts. The recovery efficiency of the analysis is 50% for both cysts and oocysts (seeded with 100 and only 50 detected). The sample, therefore, contained 60 cysts and 20 oocysts.

Throughout this specification and the claims which follow, unless the context requires otherwise, the word "comprise", or variations such as "comprises" or "comprising", will be understood to imply the inclusion of a stated integer or step or group of integers or steps but not the exclusion of any other integer or step or group of integers or steps.

### References

Anon. Method 1623: *Cryptosporidium* and *Giardia* in Water by Filtration/IMS/FA. United States Office of Water EPA-821-R-99-006. Environmental Protection Agency Washington, DC 20460 April 1999.
Yeomans, C.; Porteous, F.; Paterson, E.; Meharg, A and Killham, K., 1999. Assessment of lux-marked *Pseudomonas fluorescens* for reporting on organic carbon compounds. *FEMS Microbiology Letters,* Volume 176, Issue 1, Pages 79-83
Gröndahl, G.; Johannisson, A. and Jensen-Waern. M., 1997. Opsonic effect of equine plasma from different donors. *Veterinary Microbiology,* Volume 56, Issue 3-4, Pages 227-235
Doi. N. and Yanagawa, H., 1999. Design of generic biosensors based on green fluorescent proteins with allosteric sites by directed evolution, *FEBS Letters,* Volume 453, Issue 3, Pages 305-307
Chaka, W.; Scharringa, J.; Verheul, A.F.M.; Verhoef, J.; Van Strijp, A.G.; Hoepelman, I.M., 1995. Quantitative analysis of phagocytosis and killing of cryptococcus neoformans by human peripheral blood mononuclear cells by flow cytometry, *Clinical and Diagnostic Laboratory Immunology,* Volume 2, Issue 6, Pages 753-759
Haugland, R.P., 1998. *Handbook of fluorescent probes.* Molecular Probes, Eugene, USA (www.probes.com)
Shapiro, H.M., 1995. *A practical guide to flow cytometry,* third edition. A.R. Liss, New York.
Vesey, G., Hutton, P.E., Champion, A.C., Ashbolt, N.J., Williams, K.L., Warton, A., and Veal, D.A., 1994. Application of flow cytometric methods for the routine detection of *Cryptosporidium* and *Giardia* in water. *Cytometry,* 16, 1-6.
Vesey, G.; Narai, J; Ashbolt, N., Williams, K.L. and Veal, D.A. 1994. Detection of specific microorganisms in environmental samples using flow cytometry. In: *Methods in Cell Biology, Volume 42 -Flow Cytometry Second Edition.* ed. Darzynkiewicz, Z., Robinson; J.P. and Crissman, H.A. pp.489-522, Academic Press Inc., New York.

## Claims

1. A method for calibrating or determining accuracy of a microbial assay, comprising:
(a) providing an internal quality control standard to a sample suspected of containing a microorganism type, the standard comprising a defined quantum of a modified form of the microorganism type, wherein the modified microorganism comprises a detectable tag such that the modified microorganism can be differentiated from the corresponding unmodified microorganism In a sample being assayed;
(b) carrying out the assay so as to detect and determine the number of unmodified microorganisms and the number of modified microorganisms present in the sample; and
(c) comparing the number of modified microorganisms detected by the assay with the defined quantum of modified microorganisms provided to the sample so as to calibrate or determine the accuracy of the assay, wherein the ratio of the number of detected modified microorganisms to the defined quantum of modified microorganism added to the sample is indicative of the accuracy of the assay for the microorganism.

2. The method according to claim 1 wherein the Internal quality control standard comprises at least two types of modified microorganism so that the assay can be calibrated for each microorganism type.

3. The method according to claim 1 or 2 wherein the microorganism is selected from the group consisting of bacterium, protozoa, yeast, fungi, and virus.

4. The method according to claim 3 wherein the microorganism is selected from the group consisting of *Cryptosporidium, Giardia, Cyclospora, Toxoplasma, Eimeria, Legionella, Samonella, Leptospirosis, Escherichia, Saccharomyces, Clostridium, Vibrio, Pseudomonas,* human immunodeficiency virus, Norwalk virus, and herpes simplex virus.

5. The method according to claim 4 wherein the microorganism is *Cryptosporidium.*

6. The method according to claim 4 wherein the microorganism is *Giardia.*

7. The method according to any one of claims 1 to 6 wherein the modified microorganism is inactivated such that the microorganism is not viable.

8. The method according to any one of claims 1 to 7 wherein the modified microorganism comprises a detectable tag attached to surface molecules on the microorganism or resides inside the microorganism.

9. The method according to claim 1 wherein the modified microorganism is a microorganism that is detectably tagged by modification of the DNA or RNA, which modification alters the chemical and/or physical properties of the microorganism.

10. The method according to claim 8 wherein the modified microorganism comprises a detectable tag attached to its outer surface.

11. The method according to any one of claims 1 to 10 wherein the detectable tag alters a chemical property of the microorganism, alters a physical property of the microorganism, or alters both a chemical property and a physical property of the microorganism in a manner which allows the modified microorganism to be differentiated from a corresponding unmodified microorganism present in a sample being assayed.

12. The method according to any one of claims 1 to 11 wherein the detectable tag is a fluorescent tag.

13. The method according to claim 12 wherein the fluorescent tag is a red, blue or green fluorochrome.

14. The method according to any one of claims 1 to 13 wherein the predetermined quantum of modified microorganism Is in a diluent of defined volume.

15. The method according to any one of claims 1 to 14 wherein the assay is for detection of microorganisms In samples selected from the group consisting of water or other potable liquid, food, blood, sputum, mucous, urine and cerebrospinal fluid.

16. The method according to claim 15 wherein the potable fluid is fruit juice, wine, beer, milk, or cider, and the food is poultry, beef, eggs, cheese, or preserved meats.

17. The method according to claim 15 wherein the assay for detection of microorganisms in water is by microscopy.

## Patentansprüche

1. Verfahren zur Kalibrierung und Bestimmung der Genauigkeit eines mikrobiellen Tests, umfassend:
(a) Hinzufügen eines Standards für die interne Qualitätskontrolle zu einer Probe, die in Verdacht steht, eine Mikroorganismenart zu enthalten, wobei der Standard eine definierte Menge einer modifizierten Form der Mikroorganismenart umfasst und wobei der modifizierte Mikroorganismus eine nachweisbare Markierung aufweist, so dass der modifizierte Mikroorganismus von dem entsprechenden nicht modifizierten Mikroorganismus in der zu untersuchenden Probe unterschieden werden kann,
(b) Durchführen des Tests zum Nachweis und zur Bestimmung der Anzahl der nicht modifizierten Mikroorganismen und der Anzahl der modifizierten Mikroorganismen, die in der Probe vorliegen, und
(c) Vergleichen der Anzahl der durch den Test nachgewiesenen modifizierten Mikroorganismen mit der definierten Menge modifizierter Mikroorganismen, die zu der Probe zugegeben wurden, um den Test zu kalibrieren oder dessen Genauigkeit zu bestimmen, wobei das Verhältnis der Anzahl der nachgewiesenen modifizierten Mikroorganismen zu der definierten Menge modifizierter Mikroorganismen, die der Probe hinzugefügt wurden, die Genauigkeit des Tests auf den Mikroorganismus anzeigt.

2. Verfahren nach Anspruch 1, bei dem der Standard für die interne Qualitätskontrolle wenigstens zwei Arten modifizierter Mikroorganismen umfasst, so dass der Test für jede der Mikroorganismenarten kalibriert werden kann.

3. Verfahren nach Anspruch 1 oder 2, bei dem der Mikroorganismus aus der Gruppe ausgewählt ist, die aus Bakterien, Protozoen, Hefen, Pilzen und Viren besteht.

4. Verfahren nach Anspruch 3, bei dem der Mikroorganismus aus der Gruppe ausgewählt ist, die aus *Cryptosporidium, Giardia, Cyclospora, Toxoplasma, Eimeria, Legionella, Salmonella, Leptospirosis, Escherichia, Saccharomyces, Clostridium, Vibrio, Pseudomonas,* humanem Immundefizienzvirus, Norwalk-Virus und Herpes-simplex-Virus besteht.

5. Verfahren nach Anspruch 4, bei dem der Mikroorganismus *Cryptosporidium* ist.

6. Verfahren nach Anspruch 4, bei dem der Mikroorganismus *Giardia* ist.

7. Verfahren nach einem der Ansprüche 1 bis 6, bei dem der modifizierte Mikroorganismus inaktiviert ist, so dass er nicht lebensfähig ist.

8. Verfahren nach einem der Ansprüche 1 bis 7, bei dem der modifizierte Mikroorganismus eine nachweisbare Markierung umfasst, die an Oberflächenmolekülen des Mikroorganismus angefügt ist oder sich im Inneren des Mikroorganismus befindet.

9. Verfahren nach Anspruch 1, bei dem der modifizierte Mikroorganismus ein durch Modifikation der DNA oder RNA nachweisbar markierter Mikroorganismus ist, wobei die chemischen und/oder physikalischen Eigenschaften des Mikroorganismus durch diese Modifikation verändert werden.

10. Verfahren nach Anspruch 8, bei dem der modifizierte Mikroorganismus eine nachweisbare Markierung umfasst, welche an dessen Oberfläche angefügt ist.

11. Verfahren nach einem der Ansprüche 1 bis 10, bei dem durch die nachweisbare Markierung eine chemische Eigenschaft des Mikroorganismus, eine physikalische Eigenschaft des Mikroorganismus, oder sowohl eine chemische Eigenschaft als auch eine physikalische Eigenschaft des Mikroorganismus in einer Weise verändert wird, die es ermöglicht, in der zu untersuchenden Probe den modifizierten Mikroorganismus von einem entsprechenden nicht modifizierten Mikroorganismus zu unterscheiden.

12. Verfahren nach einem der Ansprüche 1 bis 11, bei dem die nachweisbare Markierung eine Fluoreszenzmarkierung ist.

13. Verfahren nach Anspruch 12, bei dem die Fluoreszenzmarkierung rotes, blaues oder grünes Fluorochrom ist.

14. Verfahren nach einem der Ansprüche 1 bis 13, bei dem die vorbestimmte Menge des modifizierten Mikroorganismus in einem Verdünnungsmittel vorliegt, dessen Volumen definiert ist.

15. Verfahren nach einem der Ansprüche 1 bis 14, bei welchem der Test dem Nachweis von Mikroorganismen in Proben dient, welche aus der Gruppe ausgewählt sind, die aus Wasser oder einer anderen trinkbaren Flüssigkeit, Nahrungsmitteln, Blut, Sputum, Mukus, Urin und Zerebrospinalflüssigkeit besteht.

16. Verfahren nach Anspruch 15, bei dem die trinkbare Flüssigkeit Fruchtsaft, Wein, Bier, Milch oder Obstmost/wein und das Nahrungsmittel Geflügel, Rindfleisch, Ei, Käse oder haltbar gemachtes Fleisch ist.

17. Verfahren nach Anspruch 15, bei dem der Test zum Nachweis von Mikroorganismen in Wasser durch Mikroskopie erfolgt.

## Revendications

1. Procédé pour le calibrage ou la détermination de la précision d'une analyse microbienne, comprenant :
(a) la fourniture d'une norme de contrôle de qualité interne pour un échantillon suspecté de contenir un type de microorganisme, la norme comprenant un quantum défini d'une forme modifiée du type de microorganisme, dans lequel le microorganisme modifié comprend une marque détectable telle que le microorganisme modifié puisse être différencié du microorganisme non modifié correspondant dans un échantillon qui est analysé ;
(b) la réalisation de l'analyse de façon à détecter et à déterminer le nombre de microorganismes non modifiés et le nombre de microorganismes modifiés présents dans l'échantillon ;
et
(c) la comparaison du nombre de microorganismes modifiés détecté au moyen de l'analyse avec le quantum défini de microorganismes modifiés fourni à l'échantillon de façon à calibrer ou à déterminer la précision de l'analyse, dans laquelle le rapport du nombre de microorganismes modifiés détectés au quantum défini de microorganisme ajoutée à l'échantillon est indicative de la précision de l'analyse pour le microorganisme.

2. Procédé selon la revendication 1 dans lequel la norme de contrôle de qualité interne comprend au moins deux types de microorganisme modifié de façon à ce que l'analyse puisse être calibrée pour chaque type de microorganisme.

3. Procédé selon la revendication 1 ou 2, dans lequel le microorganisme est sélectionné parmi le groupe composé de bactéries, protozoaires, levures, champignons, et virus.

4. Procédé selon la revendication 3 dans lequel le microorganisme est sélectionné parmi le groupe composé de *Cryptosporidium, Giardia, Cyclospora, Toxoplasma, Eimeria, Legionella, Salmonella, Leptospirosis, Escherichia, Saccharomyces, Clostridium, Vibrio, Pseudomonas,* virus de l'immunodéficience humaine, virus de Norwalk, et virus de l'herpès simplex.

5. Procédé selon la revendication 4 dans lequel le microorganisme est *Cryptosporidium.*

6. Procédé selon la revendication 4 dans lequel le microorganisme est *Giardia.*

7. Procédé selon l'une quelconque des revendications 1 à 6 dans lequel le microorganisme modifié est inactivé de telle façon que le microorganisme ne soit pas viable.

8. Procédé selon l'une quelconque des revendications 1 à 7 dans lequel le microorganisme modifié comprend une marque détectable attachée aux molécules de surface sur le microorganisme ou réside à l'intérieur du microorganisme.

9. Procédé selon la revendication 1 dans lequel le microorganisme modifié est un microorganisme qui est marqué de façon détectable au moyen d'une modification de l'ADN ou de l'ARN, laquelle modification modifie les propriétés chimiques et/ou physiques du microorganisme.

10. Procédé selon la revendication 8 dans lequel le microorganisme modifié comprend une marque détectable attachée à sa surface extérieure.

11. Procédé selon l'une quelconque des revendications 1 à 10 dans lequel la marque détectable modifie une propriété chimique du microorganisme, modifie une propriété physique du microorganisme, ou modifie à la fois une propriété chimique et une propriété physique du microorganisme d'une façon qui permet au microorganisme modifié d'être différencié d'un microorganisme non modifié correspondant présent dans un échantillon qui est analysé.

12. Procédé selon l'une quelconque des revendications 1 à 11 dans lequel la marque détectable est une marque fluorescente.

13. Procédé selon la revendication 12 dans lequel la marque fluorescente est un fluorochrome rouge, bleu ou vert.

14. Procédé selon l'une quelconque des revendications 1 à 13 dans lequel le quantum prédéterminé de microorganisme modifié est dans un diluant de volume défini.

15. Procédé selon l'une quelconque des revendications 1 à 14 dans lequel l'analyse est pour la détection de microorganismes dans des échantillons sélectionnés parmi le groupe composé de l'eau ou d'un autre liquide potable, d'aliment, sang, expectoration, mucus, urine et liquide cérébrospinal.

16. Procédé selon la revendication 15 dans lequel le liquide potable est le jus de fruit, le vin, la bière, le lait, ou le cidre, et l'aliment est la volaille, le boeuf, les oeufs, le fromage, ou des viandes en conserve.

17. Procédé selon la revendication 15 dans lequel l'analyse pour la détection de microorganismes dans l'eau est par microscopie.
